# EUROPEAN PATENT SPECIFICATION

(11) **EP 1 902 784 B1**
(45) Date of publication and mention of the grant of the patent: **04.08.2010**
(21) Application number: 07104331.9
(22) Date of filing: 16.03.2007
(51) Int. Cl.: B01L 3/00

(54) **Method of removing air bubbles from hybridization solution of microarray-coverslip assembly**
Verfahren zum Entfernen von Luftblasen aus einer Hybridisierungslösung einer Microarray-Deckglasanordnung
Procédé de suppression de bulles d'air d'une solution d'hybridation d'un ensemble de microréseau-fenêtre de protection

(30) Priority: 19.09.2006 KR 20060090466
(43) Date of publication of application: 26.03.2008
(73) Proprietor: Samsung Electronics Co., Ltd., Suwon-si, Gyeonggi-do (KR)
(72) Inventor: Lee, Myo-yong c/o Samsung Advanced Institute of Technology, Gyeonggi-do (KR)
(74) Representative: Grünecker, Kinkeldey, Stockmair & Schwanhäusser Anwaltssozietät

(56) References cited:
- EP-A- 0 785 433
- US-A1- 2002 095 073
- US-A1- 2004 241 759
- US-A1- 2005 266 582
- US-A1- 2006 246 490

## Description

### BACKGROUND OF THE INVENTION

### 1. Field of the Invention

The present invention relates to a method of removing air bubbles captured in a hybridization region of a microarray, and more particularly, to a method of removing air bubbles captured in a region of a microarray that is covered by a coverslip, which region is effective for hybridization, using magnetic particles and magnets.

### 2. Description of the Related Art

Microarrays are microchips for biological purposes of analyzing and monitoring gene expressions, gene distributions, mutations by forming an array of hundreds to hundred of thousands of probe molecules, such as DNA, DNA fragments, cDNA, oligonucleotide, RNA, RNA fragments, or the like, of which sequences are known, on the surface of a small solid substrate made of a material such as surface-modified glass, silicon, nylon or the like.

The term "probe" refers to a molecule, preferably a biomolecule, which is immobilized on the surface of the microarray substrate and that can selectively bind a specific target molecule.

A "microarray" is a collection of probe molecules attached to the surface of a solid support or substrate.

When biomolecules that can act as a probe are immobilized on the surface of a microarray and a sample to be assayed is applied to the microarray, the probes can detect specific target biomolecules that are included in a sample. The biomolecules in the sample differently bind with the probe immobilized on the surface of the microarray, depending on sequence complementarity or binding affinity, by hybridizing with a probe. Detecting and analyzing the hybridization can be obtain information of biomolecules, such as nucleic acids, that are included in the sample. In this way, microarrays can be used to obtain extensive information within a short period of time, and thus have been highlighted as an innovative technique that is useful for scientific technique research, new medicine development, clinical diagnosis, agriculture, foods, and environment field, etc.

In general, in an analysis using a microarray, hybridization comprises steps as follows: a certain amount of a hybridization solution including a sample is dropped on a slide glass with biomolecule probes, such as DNA, etc., immobilized thereon; the slide glass is covered by a coverslip to widely spread the hybridization solution; and the slide glass-coverslip assembly is placed and incubated in a chamber or in an incubator that is maintained at a predetermined temperature. There are two major problems in hybridization using a coverslip. One is the uneven distribution of a hybridization solution on a microarray causing the formation of a gradient. The other problem is air bubbles formation in the hybridization solution when a hybridization solution is applied to a microarray and then the microarray is covered by a coverslip. In the former case, the problem of uneven distribution of the hybridization solution can be solved by using a Lifterslip^{™} (Erie Scientific Company, US) that can distribute the same amount of hybridization solution on each spot of a microarray. Edges of both ends of the Lifterslip^{™} (Erie Scientific Company, US) have a thickness of 0.04-0.06 mm, and the hybridization solution is uniformly spread on the surface of the microarray under the coverslip so that the variation in the amount of the hybridization solution per spot can be reduced. In the case of air bubble formation in the hybridization solution, its severity depends on the skills of the practitioner. Specific solutions to the problem of air bubbles formation have not been found, and therefore caution must be taken not to form air bubbles in the first place. When air bubbles form after a hybridization solution is introduced on a microarray and the microarray is covered by a coverslip, the portion of the microarray, on which air bubbles form, is not fully in contact with and reacted with a sample so that hybridization cannot occur in those portions of the microarray, where air bubbles appear. As a result, some of the sample and a certain part of the microarray are wasted. In particular, when the microarray is used for clinical assays of patients, air bubbles formed in a hybridization area affect the result, and thus it may cause experimental errors in the clinical examination analysis. According to user manuals of microarray kits that are commercially available from Agilent, Corning, Telechem, or manuals that are used in national laboratories such as NIH/NHGRI, or universities, it is recommended that caution must be taken not to cause formation of air bubbles in a hybridization solution, and when air bubbles are trapped, the experiment should be proceeded, while discarding the data from the area where air bubbles formed rather than trying to remove the air bubbles. This is because removing air bubbles included in the hybridization solution beneath the coverslip is difficult, and the difficult-to-obtain sample and expensive microarrays may be wasted while trying to remove the air bubbles. Therefore, in a process of preparing hybridization, utmost caution is taken by slowly covering the microarray with the coverslip not to form air bubbles, and in the case of air bubbles that cannot be removed, the data from the microarray regions, where air bubble are formed, is discarded. However, taking into consideration the invaluable importance of sample, the time and labor consumed for preparing a sample and the expense of a microarray, the development of a method of making the most of a microarray by reducing experimental errors is required.

US 2006/246490 A1 relates to miniature integrated fluidic systems for carrying out a variety of preparative and analytical operations. For degassing purposes the chamber of such system may be provided with one or more vents or with one wall completely or substantially bounded by a hydrophobic membrane to allow the passage of dissolved or trapped gasses.

US 2005/266582 A1 relates to microfluidic devices and systems for performing chemical, biochemical and cellular arrays. The device may be provided with a gas permeable membrane functioning as an integrated degasser/de-bubbler.

EP 0 785 433 A2 concerns a test card holding samples for chemical or biological sample analysis. The card comprises a fluid passage network provided on both sides of the card to realize a maximum separation distance between adjacent growth wells. The card may further be provided with an improved bubble trap including a bubble trap passage having a restriction formed therein. The restriction prevents air bubbles in the bubble trap from migrating back into the growth wells.

US 2004/241759 A1 refers to screening of mixed population of organisms or nucleic acids and more specifically to the identification of bioactive molecules using screening techniques including high-throughput screening and capillary array platforms for screening samples. To disrupt the air-bubble and/or mix the contents of the capillary, paramagnetic beads contained within a capillary can be used which beads are magnetically moved from one location to another by magnetic fields applied in proximity of the capillary.

Therefore, the inventor of the present invention found a method of removing air bubbles included in a hybridization solution within a microarray-coverslip assembly using magnetic particles, based on experimental results showing that magnetic particles do not affect spots on a DNA microarray, thus completing the present invention.

### SUMMARY OF THE INVENTION

The present invention provides a method of removing air bubbles from a hybridization solution applied to a microarray-coverslip assembly comprising a coverslip and a microarray, using magnetic particles.

According to an aspect of the present invention, there is provided a method of removing air bubbles from a hybridization solution in a microarray-coverslip assembly using magnetic particles comprising: introducing a solution of magnetic particles between the coverslip and the microarray; applying a magnetic field to an assembly of the coverslip and the microarray; and removing air bubbles in a hybridization solution from a hybridization area of the microarray by moving the magnetic particles via the magnetic field.

In the method of the present invention, applying the magnetic field to the microarray-coverslip assembly comprises contacting at least one magnet with the downside of the microarray, the upside of the coverslip, or both the downside of the microarray and the upside of the coverslip.

Biomolecules, which are immobilized on the microarray, may be selected from the group consisting of DNA, RNA, PNA (peptide nucleic acid), LNA (locked nucleic acid), peptide and protein. Preferably, the biomolecules immobilized on the microarray are nucleic acids such as DNA, RNA, PNA, LNA, and the like, and more preferably DNA.

The microarray may use a surface-modified glass slide as a substrate.

The coverslip may be a LiferSlip^{™} (Erie Scientific Company, US).

The hybridization solution may be a 2x hybridization solution. Further, the hybridization solution used in hybridization of the microarray may comprise bovine serum albumin, a Denhart's solution, detergents and the like in order to inhibit non-specific binding between a probe and a sample. However, these additive lead to an increase in the viscosity of the hybridization solution and, as a result of the increased viscosity, air bubbles are more easily formed in the hybridization solution.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates a microarray-coverslip assembly in a state where air bubbles are captured in a hybridization solution applied to the microarray-coverslip assembly that is formed by applying the hybridization solution to a microarray and covering it with a coverslip (upper image), and the profile of microarray spots detected after hybridizing the probes of the microarray with targets (lower image);
FIG. 2 illustrates the profile and the signal strength of spots when magnetic particles are applied to a hybridization solution of a microarray-coverslip assembly;
FIG. 3 illustrates photographic images for illustrating a method of applying a magnetic field to the microarray-coverslip assembly using at least one magnet when air bubbles are captured in the hybridization area of a microarray, according to an embodiment of the present invention; and
FIG. 4 illustrates photographic images for illustrating a method of removing air bubbles from the hybridization area of a microarray, according to an embodiment of the present invention.

### DETAILED DESCRIPTION OF THE INVENTION

Hereinafter, the present invention will be described in detail by explaining embodiments of the invention with reference to the attached drawings.

In general, in hybridization analysis of a microarray using a coverslip, a hybridization solution including a sample to be analyzed is applied to a microarray with desired biomolecules immobilized thereon as a probe. Then, the microarray is covered with a coverslip to form a microarray-coverslip assembly. When the hybridization solution including the sample is not uniformly distributed on the microarray in this process, this uneven distribution may adversely affect the hybridization signals and the analysis results. When a Lifterstip^{™} (Erie Scientific Company, US) is used as the coverslip, the Lifterslip^{™} (Erie Scientific Company, US) is placed on the microarray and a hybridization solution is applied to the microarray-coverslip assembly, and thus, the hybridization solution can be uniformly distributed on the microarray. Edges of both ends of the Lifterslip^{™} (Erie Scientific Company, US) have a thickness of 0.04-0.06 mm, and due to capillary forces, the hybridization solution can be uniformly distributed on the surface of a microarray covered by the coverslip.

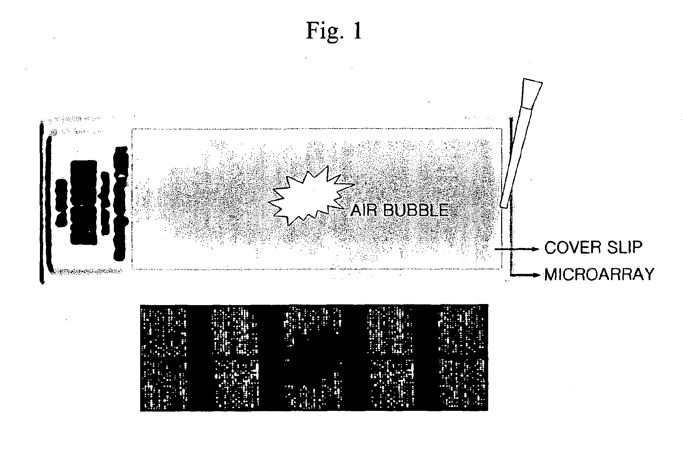

In addition, a hybridization solution is somewhat viscous. FIG. 1 illustrates a state where air bubbles are captured in a hybridization solution within a microarray-coverslip assembly that is formed by applying the hybridization solution to the microarray and covering the microarray with a coverslip. The lower image of Fig. 1 shows the resulting profile of the detected microarray probe spots. As illustrated in FIG. 1, when the hybridization solution including a sample is applied to the microarray and then the microarray is covered with a coverslip, air bubbles can be captured in the hybridization solution. Spots of the microarray, on which air bubbles are formed, cannot be effectively used for the hybridization reaction with a sample, thus only a part of the microarray can be used for detecting target molecules, and the other part of the sample and the hybridization solution are wasted unless the air bubbles are removed before carrying out the hybridization reaction. According to user manuals of commercially available microarray kits or manuals used in laboratories, extreme caution must be taken to avoid air bubble formation, and it is recommended that when air bubbles are observed underneath a coverslip, the experiment should be proceeded as planned, rather than trying to remove the air bubbles, since the effort may result in complete failure of the microarray-based experiment.

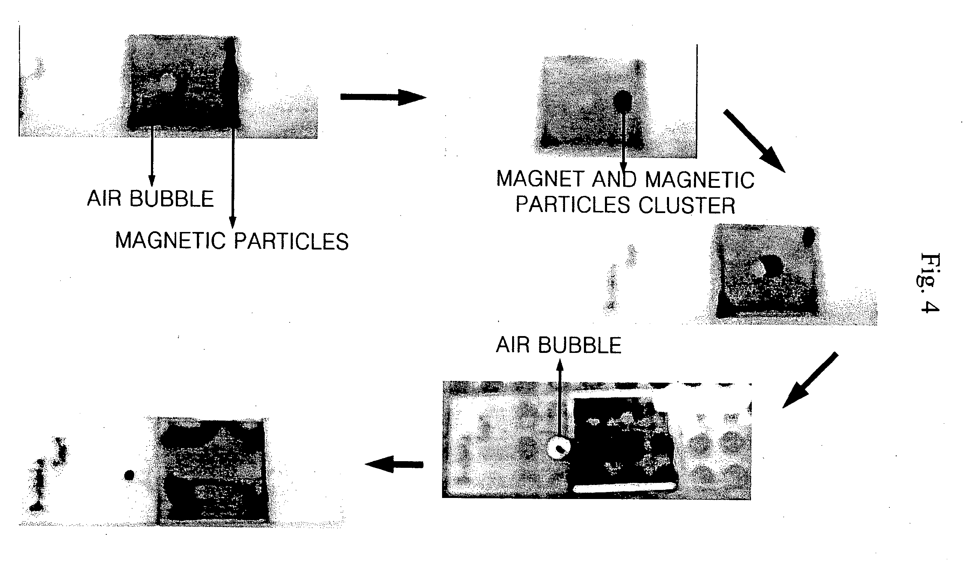

As illustrated in FIG. 4, when air bubbles are formed in a hybridization solution of a microarray-coverslip assembly, a solution of magnetic particles may be injected between the microarray and the coverslip to remedy the hybridization inhibition caused by air bubble formation.

According to an embodiment of the present invention, the magnetic particles used in the magnetic particle solution may have a diameter between 0.5 µm and 60 µm.

Further, according to an embodiment of the present invention, the surfaces of the magnetic particles are preferably negatively charged in order to avoid that negatively charge sample molecules, such as DNA, attach to the magnetic beads.

The diameter of the magnetic particles has to be such that the particles can be moved by a magnet that contacts the downside of a microarray, the upside of a coverslip or both. Therefore, the magnetic particles may have a diameter between 0.5 µm and 60 µm. To move the air bubbles by applying a magnetic field to the magnetic particles, an effective amount of magnetic particles has to be included in the magnetic particle solution. The smaller the diameter of the magnetic particles is, the more magnetic particles have to be included in the magnetic particle solution. The concentration of magnetic particles has to be 1 x 10⁶-1 x 10⁸ particles per 0.5-10 µℓ of hybridization solution.

The magnetic particle solution may be prepared by adding magnetic particles to a hybridization solution or demineralized water.

The magnetic particle solution can be injected between a coverslip and a microarray using a conventional method. For example, the tip of a micropipette containing the solution of magnetic particles can be placed onto the contact area between the coverslip and the microarray, and then the solution can be cautiously injected thereto.

A method of removing air bubbles according to an embodiment of the present invention includes removing the air bubbles in a hybridization solution from a hybridization area of the microarray by applying a magnetic field to the magnetic particles in an assembly comprising a coverslip and a microarray.

As used herein, the term "hybridization area" refers to the area on the microarray where hybridization between probes immobilized on the spots and target molecules in a sample may occur.

When magnetic particles are moved in a hybridization solution between the coverslip and the microarray of the assembly by moving magnets on the upside, downside or both sides of the assembly, air bubbles are moved out of the hybridization area, according to the motion of the magnetic particles. Thereafter, the magnetic particles are also removed from the hybridization area, and when hybridization is performed, the entire hybridization area of the microarray can be effectively used and no areas of the microarray are wasted due to air bubbles.

According to an embodiment of the present invention, the method of applying a magnetic field to the assembly of the coverslip-microarray includes contacting at least one magnet to downside, upside or both sides of the microarray assembly. Preferably, the magnet is contacted with both the downside of the microarray and the upside of the coverslip, that is, on the upside and downside of the microarray-coverslip assembly.

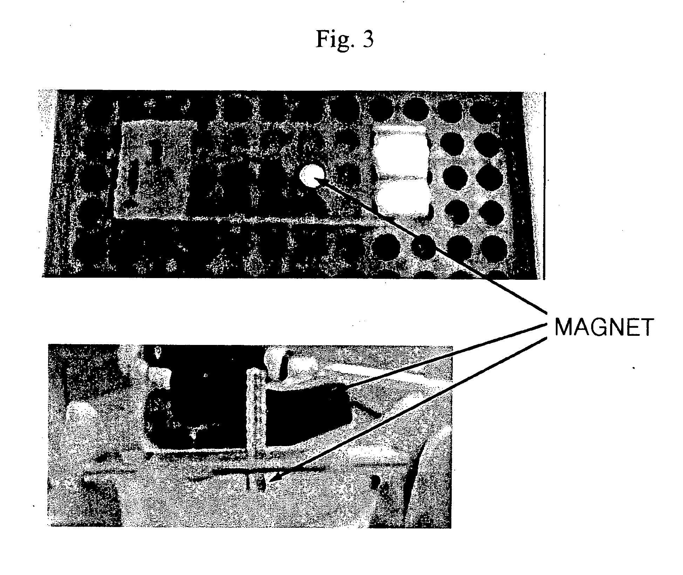

A magnetic particle solution may be injected between the microarray and the coverslip, and then magnets may be applied to the downside of the microarray or the upside of the coverslip so that the magnetic particles are magnetized. As illustrated in FIG. 3, when magnets are simultaneously applied to the downside of the microarray and the upside of the coverslip, a greater and more intensive force may be applied to the magnetic particles so that the magnetic particles in the hybridization solution can be easily moved.

A microarray kit comprising a microarray with biomolecules immobilized thereon, a coverslip, a hybridization solution, preferably a 2X hybridization solution, magnetic particles, and at least one magnet can be used for removing bubbles. Wherein the magnetic particles and the magnet are used for removing air bubbles trapped in a microarray-coverslip assembly formed when applying a hybridization solution including a sample to the microarray and then covering the microarray with the coverslip. The kit may further comprise a user manual

Various microarray kits comprising microarrays for diagnosing specific diseases or detecting genetic characteristics, and all the elements required for analysis of samples such as any reagents required for hybridization and signal detection have recently been developed. In the case of a microarray kit that performs hybridization in which the microarray is covered by a coverslip, air bubbles can be formed in a hybridization solution underneath the coverslip. To remove the air bubbles, according to the current embodiment of the present invention, magnetic particles and a magnet can be used. Therefore, when magnetic particles and at least one magnet are introduced in a microarray kit, the microarray kit can be effectively used without wasting any hybridization area and samples and experimental errors can be reduced using the method according to an embodiment of the present invention.

The microarray kit comprises a user manual, the manual which describes a method of removing air bubbles using magnetic particles and a magnet included in the microarray kit, when air bubbles are formed in a hybridization area underneath a coverslip. As stated above, air bubbles may be trapped in the hybridization solution underneath the coverslip when applying a hybridization solution including a sample to the hybridization area after a microarray-coverslip assembly is formed by covering a microarray with a coverslip.

Hereinafter, the present invention will be described in further detail with reference to the following examples. These examples are only for illustrative purposes only and are not intended to limit the scope of the present invention.

### Example 1. Effect of magnetic particles on hybridization between probe and target in a sample

The effect of magnetic particles on hybridization between a probe immobilized on a microarray and a sample was examined, when air bubbles in a hybridization solution were removed using the method according to an embodiment of the present invention.

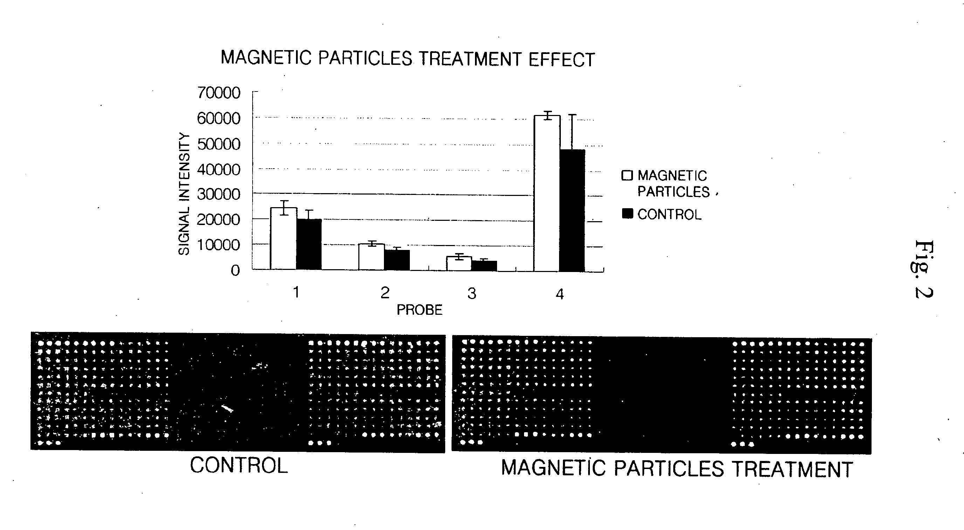

A microarray (a chip for identifying respiratory infection disease pathogen) was covered with a Lifterslip^{™} (Erie Scientific Company, US) (Erie Scientific Company, P/N 25x251-2-4823) to form a microarray-coverslip assembly. 30 µℓ of a hybridization solution (6X SSPE including 0.005% Triton X-100) including a sample was cautiously applied to the microarray to minimize the formation of air bubbles, and the hybridization solution was uniformly distributed over the entire area of the microarray between the microarray and the coverslip. Then, a solution of magnetic particles, which is a hybridization solution (6X SSPET: 0.5-10 µℓ) containing magnetic particles of 1 x 10⁷ Dynabead M-270 carboxylic acid (Dynal Inc., diameter: 2.8 µm) was injected between the microarray and the coverslip. To move the magnetic particles in the hybridization solution under the coverslip, as illustrated in FIG. 3, magnets were simultaneously applied to the downside of the microarray and the upside of the coverslip, and then moved in a desired direction for about 15 minutes. Then, the magnetic particles were moved out of the hybridization area of the microarray. Thereafter, the microarray-coverslip assembly was incubated for hybridization at 42°C for one hour and then scanned to examine the spot profile and the signal intensity (Molecular Devices Co., GenePix4000B microarray scanner). A control was obtained by performing the above experiment using the same process, except that magnetic particles were not used. The results are illustrated in FIG. 2. Referring to FIG. 2, the magnetic particles do not affect spot profile and signal intensity in the hybridization. Furthermore, signal profile of the microarray-coverslip assembly was slightly increased compared to that of the control. This may be attributable to mixing effect generated in the process of moving the magnetic particles in the hybridization solution.

### Example 2. Removal of air bubbles using magnetic particles and magnets

When a hybridization solution including a sample was introduced into a microarray that was covered by a Lifterslip^{™} (Erie Scientific Company, US) and had biomolecules immobilized thereon, air bubbles formed in the hybridization solution were removed using magnetic particles and magnets. As illustrated in FIG. 4, when air bubbles were formed in the hybridization solution included in a microarray-coverslip assembly, a solution of 1 x 10⁷ magnetic particles was injected between the microarray and the coverslip and then the magnetic particles were moved via magnets. As illustrated in FIG. 3, the magnetic particles were exposed to a magnetic field by simultaneously contacting magnets with the downside of the microarray and the upside of the coverslip. When a magnet was contacted with the downside of the microarray and the upside of the coverslip, the magnetic particles were concentrated in the area surrounding the contact with magnets. When the magnets were moved, the magnetic particles were moved according to the motion of the magnets and air bubbles were moved out of the hybridization area on the microarray accordingly. The magnets were moved for a time (within 1-2 minutes) sufficient to entirely remove the air bubbles from the hybridization area. Then, as illustrated in FIG. 4, when no air bubbles were observed, the magnetic particles were moved towards the outside of the hybridization area of the microarray. Hybridization was performed in the microarray-coverslip assembly in which air bubbles were removed. As described in Example 1, although magnetic particles were supplied to the hybridization solution in the microarray-coverslip assembly before hybridization and magnets were moved to move the magnetic particles within the hybridization solution, it did not affect hybridization between the sample and the probe on the microarray, spot profile and signal intensity. Therefore, this pre-process performed using magnetic particles and a magnet enabled the entire hybridization area of the microarray to be effectively used.

According to the present invention, in hybridization of a microarray using a coverslip, when air bubbles are captured in a hybridization solution beneath the coverslip, the air bubbles can be removed from the hybridization area using magnetic particles and magnets so that the hybridization area of the microarray can be fully utilized.

## Claims

1. A method of removing air bubbles from a hybridization solution in a microarray-coverslip assembly comprising a coverslip and a microarray using magnetic particles, the method comprising:
introducing a liquid containing magnetic particles between the coverslip and the microarray;
applying a magnetic field to the microarray-coverslip assembly; and
removing air bubbles in the hybridization solution from a hybridization area of the microarray by moving the magnetic particles via the magnetic field.

2. The method of claim 1, whereby applying a magnetic field to the microarray-coverslip assembly comprises contacting at least one magnet with the downside of the microarray or the upside of the coverslip, or both the downside of the microarray and the upside of the coverslip.

3. The method of claim 1 or 2, wherein a biomolecule selected from the group consisting of DNA, RNA, PNA, LNA, peptide and protein is immobilized on the microarray.

4. The method of any of claims 1 to 3, wherein the magnetic particles have a diameter of 0.5 µm to 60 µm.

5. The method of any of claims 1 to 4, wherein the surfaces of the magnetic particles are negatively charged.

## Patentansprüche

1. Verfahren zum Entfernen von Luftblasen aus einer Hybridisierungslösung in einer Mikroarray-Deckglasanordnung, die ein Deckglas und einen Mikroarray umfasst, mittels Verwendens von magnetischen Partikeln, wobei das Verfahren umfasst:
Einführen einer Flüssigkeit, die magnetische Partikel enthält, zwischen das Deckglas und den Mikroarray;
Anlegen eines Magnetfeldes an die Mikroarray-Deckglasanordnung; und
Entfernen von Luftblasen in der Hybridisierungslösung aus einem Hybridisierungsbereich von dem Mikroarray durch Bewegen der magnetischen Partikel mittels des Magnetfeldes.

2. Verfahren gemäß Anspruch 1, wobei das Anlegen eines Magnetfeldes an die Mikroarray-Deckglasanordnung das Kontaktieren wenigstens eines Magnetes mit der Unterseite des Mikroarray oder der Oberseite des Deckglases oder sowohl der Unterseite des Mikroarray als auch der Oberseite des Deckglases umfasst.

3. Verfahren gemäß Anspruch 1 oder 2, wobei ein Biomolekül, das ausgewählt ist aus der Gruppe bestehend aus DNA, RNA, PNA, LNA, einem Peptid und einem Protein, auf dem Mikroarray immobilisiert ist.

4. Verfahren gemäß einem der Ansprüche 1 bis 3, wobei die magnetischen Partikel einen Durchmesser vom 0,5 µm bis 60 µm aufweisen.

5. Verfahren gemäß einem der der Ansprüche 1 bis 4, wobei die Oberflächen von den magnetischen Partikeln negativ geladen sind.

## Revendications

1. Procédé pour éliminer les bulles d'air d'une solution d'hybridation dans un ensemble microréseau-lamelle de protection comprenant une lamelle de protection et un microréseau au moyen de particules magnétiques, ledit procédé consistant à :
introduire un liquide contenant des particules magnétiques entre la lamelle de protection et le microréseau ;
appliquer un champ magnétique sur l'ensemble microréseau-lamelle de protection ; et
éliminer des bulles d'air de la solution d'hybridation dans une zone d'hybridation du microréseau grâce au déplacement des particules magnétiques sous l'effet du champ magnétique.

2. Procédé selon la revendication 1, dans lequel l'application d'un champ magnétique sur l'ensemble microréseau-lamelle de protection comprend à mettre en contact au moins un aimant avec le dessous du microréseau ou avec le dessus de la lamelle de protection, ou à la fois avec le dessous du microréseau et le dessus de la lamelle de protection.

3. Procédé selon la revendication 1 ou 2, dans lequel une biomolécule choisie dans le groupe constitué d'un ADN, d'un ARN, d'un acide nucléique peptidique (PNA), d'un acide nucléique verrouillé (LNA), d'un peptide et d'une protéine est immobilisée sur le microréseau.

4. Procédé selon l'une quelconque des revendications 1 à 3, dans lequel les particules magnétiques ont un diamètre de 0,5 µm à 60 µm.

5. Procédé selon l'une quelconque des revendications 1 à 4, dans lequel les surfaces des particules magnétiques sont chargées négativement.
